Europäisches Patentamt

European Patent Office

Office européen des brevets

(11) Veröffentlichungsnummer: **0 172 408**
A2

# (12) EUROPÄISCHE PATENTANMELDUNG

(21) Anmeldenummer: 85108897.1

(22) Anmeldetag: 16.07.85

(51) Int. Cl.⁴: **B 01 D 53/14**

(30) Priorität: 24.07.84 DE 3427134

(43) Veröffentlichungstag der Anmeldung:
26.02.86 Patentblatt 86/9

(84) Benannte Vertragsstaaten:
AT BE DE FR GB IT NL SE

(71) Anmelder: BASF Aktiengesellschaft
Carl-Bosch-Strasse 38
D-6700 Ludwigshafen(DE)

(72) Erfinder: Volkamer, Klaus, Dr.
Heidelberger Ring 21
D-6710 Frankenthal(DE)

(72) Erfinder: Hefner, Werner, Dr.
Danziger Strasse 1c
D-6840 Lampertheim(DE)

(72) Erfinder: Wagner, Eckhart, Dr.
Birkenstrasse 21a
D-6701 Maxdorf(DE)

(72) Erfinder: Wagner, Ulrich, Dr.
Knospstrasse 7
D-6703 Limburgerhof(DE)

(54) Verfahren zum Entfernen von CO2 und/oder H2S aus Gasen.

(57) Die vorliegende Erfindung betrifft ein Verfahren zum Entfernen von $CO_2$ und/oder $H_2S$ aus $CO_2$ und/oder $H_2S$ enthaltenden Gasen durch eine Wäsche der Gase mit einer wäßrigen, ein Alkanolamin enthaltenden Absorptionsflüssigkeit, bei dem man die $CO_2$ und/oder $H_2S$ enthaltenden Gase in einer Absorptionsstufe mit einer 3 bis 60 Gew.% eines tertiären Alkanolamins und 1 bis 50 Gew.% eines primären Alkanolamins enthaltenden wäßrigen Absorptionsflüssigkeit behandelt, am Kopf der Absorptionsstufe die behandelten Gase abzieht, am Sumpf der Absorptionsstufe die mit $CO_2$ und/oder $H_2S$ beladene wäßrige Absorptionsflüssigkeit abzieht und zur Regenerierung in mindestens zwei Entspannungsstufen entspannt und dabei die letzte Entspannungsstufe bei Vermindertem Druck gegenüber Atmosphärendruck betreibt und die regenerierte Absorptionsflüssigkeit in die Absorptionsstufe zurückführt.

EP 0 172 408 A2

Verfahren zum Entfernen von $CO_2$ und/oder $H_2S$ aus Gasen

Die vorliegende Erfindung betrifft ein Verfahren zum Entfernen von $CO_2$ und/oder $H_2S$ aus Gasen mittels einer wäßrigen Absorptionsflüssigkeit.

Es ist bekannt, z.B. aus A. Kohl und F. Riesenfeld, Gas Purification, 3. Auflage, 1979, insbesondere Seiten 28, 33 und 34, wäßrige Lösungen von primären Aminen wie Monoethanolamin zur Entfernung von $CO_2$ und/oder $H_2S$ aus Gasen zu verwenden. Ein Nachteil der Verwendung von wäßrigen Lösungen von Monoethanolamin besteht jedoch darin, daß die wäßrigen Monoethanolamin-Lösungen, besonders bei höheren Konzentrationen, stark korrosiv wirken. Um Korrosionsschäden in den Gaswaschanlagen zu vermeiden oder zumindest auf ein erträgliches Maß zu mindern, ist es daher erforderlich, in solchen Anlagen in aufwendiger Weise Systeme von Korrosionsinhibitoren einzusetzen, wobei jedoch diese Korrosionsinhibitoren bei $H_2S$-haltigen Gasen gar nicht eingesetzt werden können. Die bekannten Verfahren zur Entfernung von $CO_2$ und/oder $H_2S$ unter Verwendung von primären Alkanolaminen wie Monoethanolamin sind daher nicht zufriedenstellend.

Es bestand daher Bedarf nach einem Verfahren zum Entfernen von $CO_2$ und/ oder $H_2S$ aus Gasen, bei dem die Nachteile der bekannten Verfahren vermieden werden können.

Es wurde nun ein vorteilhaftes Verfahren gefunden zum Entfernen von $CO_2$ und/oder $H_2S$ aus $CO_2$ und/oder $H_2S$ enthaltenden Gasen durch eine Wäsche der Gase mittels einer wäßrigen, ein Alkanolamin enthaltenden Absorptionsflüssigkeit, welches dadurch gekennzeichnet ist, daß man die $CO_2$ und/oder $H_2S$ enthaltenden Gase in einer Absorptionsstufe mit einer 3 bis 60 Gew.-% eines tertiären Alkanolamins und 1 bis 50 Gew.% eines primären Alkanolamins enthaltenden wäßrigen Absorptionsflüssigkeit behandelt, am Kopf der Absorptionsstufe die behandelten Gase abzieht, am Sumpf der Absorptionsstufe die mit $CO_2$ und/oder $H_2S$ beladene wäßrige Absorptionsflüssigkeit abzieht und zur Regenerierung in einer oder mehreren Entspannungsstufen entspannt und die regenerierte Absorptionsflüssigkeit in die Absorptionsstufe zurückführt.

In einer vorteilhaften Ausführungsform des Verfahrens wird die mit $CO_2$ und/oder $H_2S$ beladene wäßrige Absorptionsflüssigkeit zur Regenerierung in mindestens zwei Entspannungsstufen entspannt, wobei die letzte Entspannungsstufe bei vermindertem Druck gegenüber Atmosphärendruck betrieben wird.

Ste/P

In einer weiteren vorteilhaften Ausführungsform des Verfahrens mit.mindestens zwei Entspannungsstufen führt man zum Ausgleich der Wasserverluste durch in dem am Kopf der Absorptionsstufe und den Entspannungsstufen abgezogenen Gasströmen enthaltenes Wasser am Sumpf der vorletzten Entspannungsstufe eine dem Wasserverlust entsprechende Menge Wasserdampf zu.

Nach dem neuen Verfahren wird das mit $CO_2$ und/oder $H_2S$ beladene Lösungsmittel ohne Verwendung einer Ausstreifkolonne allein durch Entspannung regeneriert, so daß eine erhebliche Reduktion sowohl der Investitionskosten als auch der Energieverbrauchskosten erreicht wird. Weiter können bei dem neuen Verfahren relativ hohe Alkanolaminkonzentrationen in der Absorptionsflüssigkeit verwendet werden. Dabei kann das Auftreten von Korrosionsschäden weitgehend vermieden werden, ohne daß ein Zusatz von Korrosionsinhibitoren erforderlich wird.

Ein weiterer Vorteil des Verfahrens besteht darin, daß bei Anwendung von mindestens zwei Entspannungsstufen Wasserverluste, die in Gaswaschanlagen durch in den am Kopf der Absorptionskolonne und der Entspannungsbehälter abgezogenen Gasströmen enthaltenes Wasser entstehen, durch Zuführung einer dem Wasserverlust entsprechenden Menge Wasserdampf am Sumpf der vorletzten Entspannungsstufe ausgeglichen werden können. Durch diese Arbeitsweise kann neben der Regelung des Wasserhaushaltes der Gaswaschanlage gleichzeitig der Wärmehaushalt der Gaswaschanlage geregelt werden, so daß ein für die Regelung des Wärmehaushaltes in der Gaswaschanlage vorhandener Wärmetauscher kleiner ausgeführt werden oder gegebenenfalls ganz wegfallen kann. Durch das Betreiben der letzten Entspannungsstufe bei vermindertem Druck wird eine regenerierte Absorptionsflüssigkeit mit einem geringerem $CO_2$- und/oder $H_2S$-Gehalt erhalten, so daß geringere Mengen an Absorptionsflüssigkeit im Kreis geführt werden müssen, wodurch sich entsprechende Einsparungen bei den Energieverbräuchen ergeben.

In einer weiteren bevorzugten Ausführungsform des Verfahrens wird bei Anwendung von mindestens zwei Entspannungsstufen der verminderte Druck in der letzten Entspannungsstufe mittels eines Wasserdampfstrahlers erzeugt. Dabei kann es vorteilhaft sein, daß man das am Kopf der letzten Entspannungsstufe abgezogene Gas zusammen mit dem Wasserdampf, mit dem der Wasserdampfstrahler betrieben wird, am Sumpf der vorletzten Entspannungsstufe zuführt.

Als nach dem erfindungsgemäßen Verfahren zu behandelnde Gase kommen beispielsweise Kohlevergasungsgase, Synthesegase, Koksofengase, Raffineriegase oder Erdgase in Betracht. Vorteilhaft wird das Verfahren zum Entfernen von $CO_2$ und/oder $H_2S$ aus Erdgasen verwendet, die neben Methan noch höhere Kohlenwasserstoffe enthalten. Bei diesen höheren Kohlenwasserstoffen handelt es sich im allgemeinen um $C_2$- bis $C_{30}$-Kohlenwasserstoffe, vorzugsweise $C_2$- bis $C_{20}$-Kohlenwasserstoffe, insbesondere $C_2$- bis $C_{12}$-Kohlenwasserstoffe, die in der Regel aliphatisch sind, z.B. Ethan, Propan, Isobutan, n-Butan, Isopentan, n-Pentan, die Hexane, Heptane, Octane, Nonane, Decane und die höheren Homologen. Die höhere Kohlenwasserstoffe können neben den aliphatischen Kohlenwasserstoffen auch noch aromatische Kohlenwasserstoffe wie Benzol enthalten. Im allgemeinen beträgt der Gehalt der Erdgase an den höheren Kohlenwasserstoffen 0,1 bis 40 Mol.%, vorzugsweise 0,5 bis 30 Mol.%, insbesondere 1 bis 20 Mol.%.

Die Gase weisen im allgemeinen einen $CO_2$-Gehalt von 1 bis 90 Mol.%, vorzugsweise 2 bis 90 Mol.%, insbesondere 5 bis 60 Mol.% auf. Neben dem $CO_2$ können die Gase als weiteres Sauergas $H_2S$ enthalten oder sie können $H_2S$ allein enthalten, z.B. in Mengen von wenigen Mol.-ppm, beispielsweise 1 Mol.-ppm bis 50 Mol.%, vorzugsweise 10 Mol.-ppm bis 40 Mol.%.

Als Lösungsmittel wird für das erfindungsgemäße Verfahren eine wäßrige Absorptionsflüssigkeit verwendet, die 3 bis 60 Gew.%, in der Regel 5 bis 60 Gew.%, im allgemeinen 10 bis 60 Gew.%, zweckmäßig 20 bis 50 Gew.%, vorzugsweise 25 bis 45 Gew.%, insbesondere 25 bis 35 Gew.% eines ter-tiären Alkanolamins und 1 bis 50 Gew.%, zweckmäßig 5 bis 40 Gew.%, vorzugsweise 10 bis 30 Gew.%, insbesondere 15 bis 20 Gew.% eines primären Alkanolamins enthält. Geeignete tertiäre Alkanolamine sind beispielsweise Triisopropanolamin, Methyldiisopropanolamin, Dimethylisopropanolamin, Diethylethanolamin, vorzugsweise Triethanolamin, insbesondere Methyldiethanolamin. Als primäre Alkanolamine kommen z.B. 2-2"-Aminoethoxy-ethanol und vorzugsweise Monoethanolamin in Betracht.

Die das tertiäre Alkanolamin und primäre Alkanolamin enthaltende wäßrige Absorptionsflüssigkeit kann zusätzlich noch ein physikalisches Lösungsmittel enthalten. Geeignete physikalische Lösungsmittel sind beispielsweise N-Methylpyrrolidon, Tetramethylensulfon, Methanol, Oligoethylenglykoldialkylether wie Oligoethylenglykolmethylisopropylether (SEPASOLV MPE), Oligoethylenglykoldimethylether (SELEXOL). Das physikalische Lösungsmittel ist in der Absorptionsflüssigkeit im allgemeinen in Mengen von 1 bis 60 Gew.%, vorzugsweise 10 bis 50 Gew.%, insbesondere 20 bis 40 Gew.% enthalten.

Das erfindungsgemäße Verfahren wird in der Weise durchgeführt, daß das $CO_2$ und/oder $H_2S$ enthaltende Gas zunächst in einer Absorptionsstufe mit der erfindungsgemäß zu verwendenden Absorptionsflüssigkeit behandelt wird. Dabei werden in der Absorptionszone im allgemeinen Temperaturen von 30 bis 90°C, vorzugsweise 35 bis 80°C, insbesondere 40 bis 60°C angewendet. Im allgemeinen werden in der Absorptionszone Drücke von 5 bis 110 bar, vorzugsweise 10 bis 100 bar, insbesondere 20 bis 90 bar angewendet. Als Absorptionszone wird zweckmäßig eine Absorptionskolonne verwendet, im allgemeinen eine Füllkörperkolonne oder eine mit Böden ausgestattete Kolonne. Zweckmäßig wird das zu behandelnde Gas am Sumpf und die Absorptionsflüssigkeit am Kopf der Absorptionskolonne zugeführt, wobei die sauren Gase $CO_2$ und/oder $H_2S$ im Gegenstrom ausgewaschen werden.

Während gegebenenfalls vorhandenes $H_2S$ zweckmäßig weitgehend, im allgemeinen auf $H_2S$-Gehalte im behandelten Gas von höchstens 120 Mol.-ppm, vorzugsweise höchstens 10 Mol.-ppm, insbesondere höchstens 3 Mol.-ppm ausgewaschen wird, kann es vorteilhaft sein, das $CO_2$ im Gas soweit auszuwaschen, daß die $CO_2$-Gehalte im behandelten Gas höchstens etwa 0,5 bis 6, vorzugsweise 0,5 bis 5, insbesondere 1 bis 4 Mol.% betragen. Das behandelte Gas wird zweckmäßig am Kopf der Absorptionszone, zweckmäßig an einem Punkt, der oberhalb der Zuführung der Absorptionsflüssigkeit liegt, abgezogen. Die mit den Sauergasen $CO_2$ und/oder $H_2S$ beladene Absorptionsflüssigkeit wird zweckmäßig am Sumpf der Absorptionszone abgezogen.

Anschließend wird die beladene Absorptionsflüssigkeit in einer oder mehreren Entspannungsstufen, im allgemeinen in mindestens 2, zweckmäßig 2 bis 5, vorzugsweise 2 oder 3, Entspannungsstufen regeneriert. Dabei wird in einer Ausführungsform des Verfahrens in der letzten Entspannungsstufe bzw., falls nur eine Entspannungsstufe verwendet wird, in dieser einzelnen Entspannungsstufe zweckmäßig auf einen Druck von etwa 1 bis 3 bar, vorzugsweise 1 bis 1,8 bar, insbesondere 1 bis 1,5 bar entspannt. Es kann auch zweckmäßig sein, die letzte Entspannungsstufe bzw. die einzelne Entspannungsstufe unter vermindertem Druck gegenüber Atmosphärendruck, z.B. bei Drucken von 0,3 bis etwa 1 bar, vorzugsweise 0,5 bis etwa 1 bar, insbesondere von 0,7 bis etwa 1 bar, zu betreiben. In einer bevorzugten Ausführungsform des Verfahrens wird die mit $CO_2$ und/oder $H_2S$ beladene wäßrige Absorptionsflüssigkeit zur Regenerierung in mindestens zwei Entspannungsstufen entspannt und dabei die letzte Entspannungsstufe bei vermindertem Druck gegenüber Atmosphärendruck betrieben und gegebenenfalls gleichzeitig zum Ausgleich der Wasserverluste durch in den am Kopf der Absorptionsstufe und den Entspannungsstufen abgezogenen Gasströmen enthaltenes Wasser am Sumpf der vorletzten Entspannungsstufe eine dem Wasserverlust entsprechende Menge Wasserdampf zugeführt. Vorzugsweise wird dabei in der letzten Entspannungsstufe ein Druck von 0,3 bis etwa 1 bar, vorzugsweise 0,5 bis etwa 1 bar, insbesondere 0,7 bis etwa 1 bar aufrechterhalten. Der verminderte Druck kann in der letzten Entspannungsstufe beispielsweise unter Verwendung eines zur Vakuumerzeugung üblicherweise verwendeten Apparates, z.B. eines mechanischen Vakuumerzeugungsapparates, wie Vakuumpumpen oder vorzugsweise Verdichter, z.B. Schraubenverdichter, Zentrifugalverdichter, oder eines Wasserdampfstrahlers eingestellt werden. Mit besonderem Vorteil wird der verminderte Druck in der
letzten Entspannungsstufe mittels eines mechanischen Vakuumerzeugungsapparates und eines Wasserdampfstrahlers, die hintereinandergeschaltet
sind, erzeugt.

Zum Ausgleich der Wasserverluste, die bei dem Verfahren durch in den am
Kopf der Absorptionsstufe und den Entspannungsstufen abgezogenen Gasströmen enthaltenes Wasser entstehen, wird bei der Arbeitsweise mit mindestens zwei Entspannungsstufen zweckmäßig am Sumpf der vorletzten Entspannungsstufe eine dem Wasserverlust entsprechende Menge Wasserdampf
zugeführt. In der Regel wird das in den abgezogenen Gasströmen enthaltene
Wasser im wesentlichen als Wasserdampf abgezogen. Dem Sumpf der vorletzten Entspannungsstufe kann Niederdruck-, Mitteldruck- oder Hochdruckdampf, d.h. z.B. 1,5- bis 100-bar-Dampf zugeführt werden. Vorzugsweise
verwendet man Dampf im niederen Druckbereich, z.B. 1,5- bis 10-bar-Dampf,
vorteilhaft 1,5- bis 5-bar-Dampf, da dieser Niederdruckdampf im allgemeinen wohlfeil zur Verfügung steht.

Das am Kopf der letzten Entspannungsstufe abgezogene Gas kann an die Atmosphäre abgegeben werden oder, falls es noch $H_2S$ enthält, durch Oxidation
des $H_2S$, z.B. in einer Claus-Anlage, aufgearbeitet werden. In einer vorteilhaften Ausführungsform des Verfahrens mit mindestens zwei Entspannungsstufen wird der verminderte Druck in der letzten Entspannungsstufe mittels eines Wasserdampfstrahlers erzeugt und dabei zweckmäßig das
am Kopf der letzten Entspannungsstufe abgezogene Gas zusammen mit dem
Wasserdampf, mit dem der Wasserdampfstrahler betrieben wird, am Sumpf der
vorletzten Entspannungsstufe zugeführt.

Falls der Wasserdampf zum Betrieb des Wasserdampfstrahlers am Sumpf der
vorletzten Entspannungsstufe zugeführt wird, wird der Wasserdampfstrahler
zweckmäßig mit einer solchen Menge Wasserdampf betrieben, wie sie zum
Ausgleich der Wasserverluste des Verfahrens erforderlich ist. Es ist jedoch auch möglich, den Wasserdampfstrahler mit einer geringeren als zum
Ausgleich der Wasserverluste entsprechenden Wasserdampfmenge zu betreiben
und am Sumpf der vorletzten Entspannungsstufe zusätzlich die zum Ausgleich der Wasserverluste noch fehlende Wasserdampfmenge zuzuführen. Zum
Betrieb des Wasserdampfstrahlers kann Mitteldruck- oder Hochdruckdampf
verwendet werden. Vorzugsweise verwendet man Dampf in mittlerem Druckbereich, z.B. 5- bis 20-bar-Dampf, vorzugsweise 5- bis 10-bar-Dampf.

Die vorletzte Entspannungsstufe wird bei der Arbeitsweise mit mindestens zwei Entspannungsstufen und Betrieb der letzten Entspannungsstufe bei vermindertem Druck zweckmäßig bei einem Druck von etwa 1 bis 5 bar, vorzugsweise 1 bis 2,5 bar, insbesondere 1 bis 1,5 bar betrieben.

Für die Entspannung werden zweckmäßig Entspannungsbehälter, die z.B. auch als Kolonnen gestaltet sein können, verwendet. Diese Entspannungsbehälter können frei von besonderen Einbauten sein. Es können jedoch auch mit Einbauten, z.B. mit Füllkörpern ausgestattete Kolonnen verwendet werden.

In der Regel wird zum Ausgleich von verfahrensbedingten Wärmeverlusten, die beispielsweise durch die Entspannungsverdampfung bewirkt werden, dem Verfahren Wärme zugeführt. Dies erfolgt zweckmäßig in einer vor die letzte Entspannungsstufe bzw., falls nur eine Entspannungsstufe verwendet wird, vor diese einzelne Entspannungsstufe geschalteten Wärmeaustauschzone, in der die beladene Absorptionsflüssigkeit vor der Entspannung in der letzten bzw. einzelnen Entspannungsstufe erwärmt wird. In der Regel wird die Absorptionsflüssigkeit in der Austauschzone um maximal 20°C erwärmt, wobei die Absorptionsflüssigkeit im allgemeinen auf eine Temperatur von höchstens 90°C, in der Regel von höchstens 95°C erwärmt wird. Für die Wärmeaustauschzone werden im allgemeinen Wärmetauscher, z.B. ein Röhrenwärmetauscher, verwendet.

Die Sauergase $CO_2$ und/oder $H_2S$ werden zweckmäßig am Kopf der letzten Entspannungsstufe bzw., falls das am Kopf der letzten Entspannungsstufe abgezogene Gas zusammen mit dem Wasserdampf für den Betrieb des Wasserdampfstrahlers am Sumpf der vorletzten Entspannungsstufe zugeführt wird, am Kopf der vorletzten Entspannungsstufe abgezogen. Die am Sumpf der letzten Entspannungsstufe abgezogene regenerierte Absorptionsflüssigkeit wird in die Absorptionsstufe zurückgeführt.

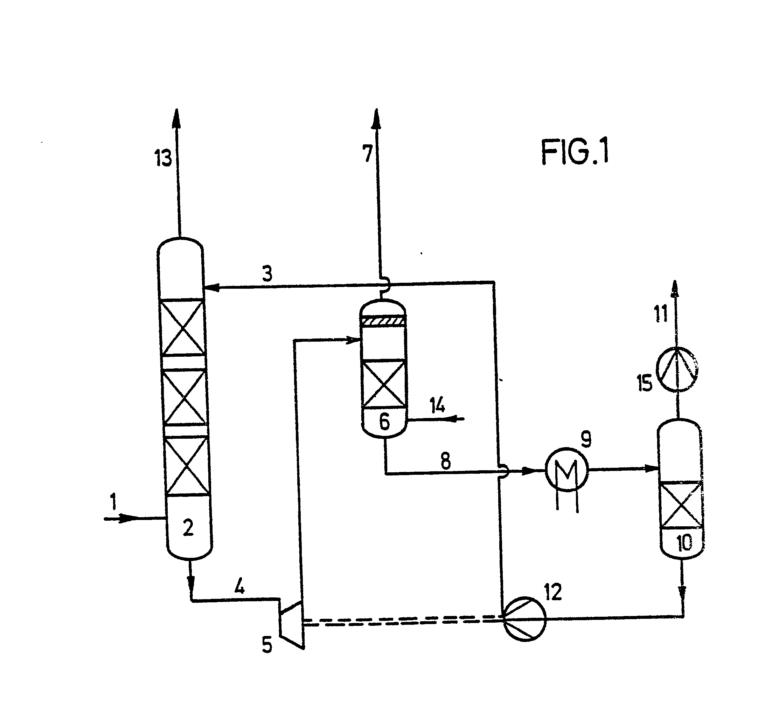

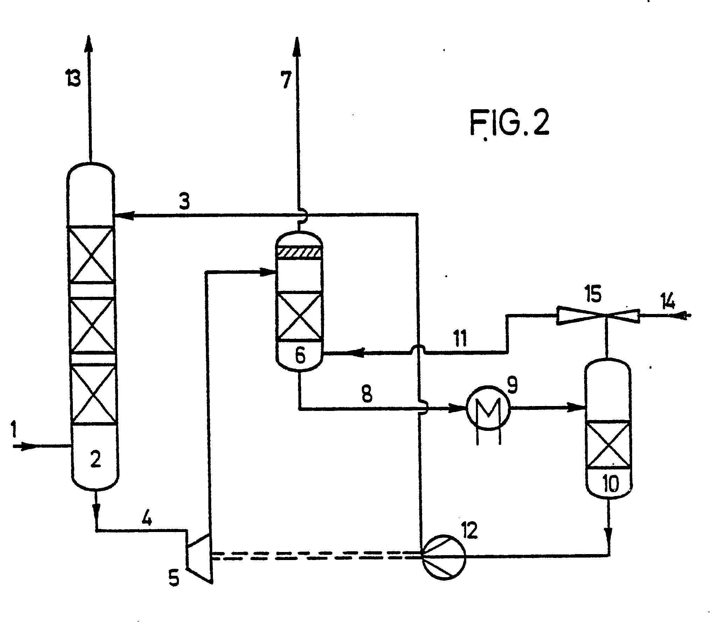

Nachfolgend werden weitere Einzelheiten der Erfindung anhand von zwei Ausführungsbeispielen, deren Verfahrensablauf in den Figuren 1 und 2 schematisch dargestellt ist, erläutert.

Gemäß Figur 1 wird ein $CO_2$ und/oder $H_2S$ enthaltendes Gas, z.B. ein höhere Kohlenwasserstoffe, z.B. aliphatische $C_2$- bis $C_{10}$-Kohlenwasserstoffe enthaltendes Erdgas, über Leitung 1 unter Druck in den Sumpf der Absorptionskolonne 2 gegeben. Gleichzeitig wird über Leitung 3 wäßrige Absorptionsflüssigkeit mit einem Gehalt von 30 Gew.% Methyldiethanolamin- und 20 Gew.% Monoethanolamin auf den Kopf der Absorptionskolonne gegeben. Die Absorptionsflüssigkeit, die im Gegenstrom zum Gas geführt wird, belädt sich mit den sauren Gasen $CO_2$ und/oder $H_2S$, und die beladene Absorptions-

flüssigkeit wird über Leitung 4 am Sumpf der Absorptionskolonne abgezogen. Das gewaschene Gas wird über Leitung 13 am Kopf der Absorptionskolonne abgezogen. Der beladene Absorptionsflüssigkeitsstrom 4 wird anschließend in einen Entspannungsverdampfungsbehälter 6 entspannt, beispielsweise über ein Ventil oder vorzugsweise über eine Entspannungsturbine 5. Hierbei wird ein Kohlenwasserstoff und $CO_2$ enthaltendes Zwischenentspannungsgas aus der Absorptionsflüssigkeit freigesetzt, das über Leitung 7 am Kopf des Entspannungsbehälters 6 abgezogen wird. In den Sumpf des Entspannungsbehälters 6 wird gegebenenfalls zum Ausgleich der Wasserverluste des Systems über Leitung 14 Wasserdampf, z.B. 2,5 bar-Niederdruckdampf, eingeleitet. Am Boden des Entspannungsbehälters 6 wird die teilentspannte Absorptionsflüssigkeit über Leitung 8 abgezogen, in Wärmetauscher 9 erwärmt, z.B. um 1 bis 15°C, und die erwärmte Absorptionsflüssigkeit wird in einen zweiten Entspannungsbehälter 10 entspannt, in dem z.B. mittels der Vakuumpumpe 15 ein Druck von z.B. 0,5 bar bis weniger als Atmosphärendruck aufrecht erhalten wird. Hierbei wird ein $CO_2$-reiches Entspannungsgas, z.B. mit einer $CO_2$-Konzentration von 98 Mol.%, freigesetzt, das über Leitung 11 am Kopf des Entspannungsbehälters 10 abgezogen wird. Die am Sumpf des Entspannungsbehälters 10 abgezogene regenerierte Absorptionsflüssigkeit wird mit Hilfe einer Umlaufpumpe 12 zum Kopf der Absorptionskolonne 2 zurückgeführt.

In einem weiteren beispielhaften Ausführungsbeispiel (vgl. Fig. 2) wird wie im ersten Ausführungsbeispiel verfahren, wobei jedoch anstelle einer Vakuumpumpe der Dampfstrahler 15 zur Erzeugung des verminderten Drucks im zweiten Entspannungsbehälter 10 verwendet wird, dem über Leitung 14 z.B. eine solche Menge Wasserdampf zugeführt wird, wie sie zum Ausgleich der Wasserverluste des Systems erforderlich ist. Das am Kopf des Entspannungsbehälters 10 abgezogene Gas wird zusammen mit dem Wasserdampf, mit dem der Wasserdampfstrahler 15 betrieben wird, am Sumpf des ersten Entspannungsbehälters 6 zugeführt.

Das folgende Beispiel veranschaulicht die Erfindung.

Beispiel

Man verwendete eine Gaswaschanlage, in der sich anschließend an eine Absorptionskolonne drei hintereinandergeschaltete Entspannungsbehälter fanden. In der Absorptionskolonne wurden 2,23 kmol/h eines $CO_2$-haltigen Erdgases mit einer wäßrigen Absorptionsflüssigkeit, die 15 Gew.% Monoethanolamin und 35 Gew.% Methyldiethanolamin enthielt, bei einem Druck von 70 bar gewaschen.

Das zu reinigende Gas hatte folgende Zusammensetzung:

$CO_2$                                    10,0 Mol.%

$CH_4$                                    75,0 Mol.%

höhere Kohlenwasserstoffe

($C_2$- bis $C_{12}$-Kohlenwasserstoffe)   15,0 Mol.%

Die Temperatur des Absorptionsmittels im Zulauf zur Absorptionskolonne betrug 60°C. Der $CO_2$-Gehalt im gewaschenen Gas betrug weniger als 2 Mol.%. Das die Absorptionskolonne verlassende beladene Waschmittel wurde in einem ersten Entspannungsbehälter auf einen Druck von 30 bar entspannt. Hierbei wurden 4,2 mol/h eines kohlenwasserstoffreichen Zwischenentspannungsgases mit einer $CO_2$-Konzentration von weniger als 10 Mol.% aus der Lösung freigesetzt und am Kopf des ersten Entspannungsbehälters abgezogen. Die teilentspannte Absorptionsflüssigkeit wurde anschließend in einem Wärmetauscher erwärmt. Die erwärmte Absorptionsflüssigkeit wurde in einem zweiten Entspannungsbehälter auf 1,5 bar entspannt. Hierbei wurden 0,1 kmol/h eines $CO_2$-reichen Entspannungsgases aus der Adsorptionsflüssigkeit entgast.

Die am Boden des zweiten Entspannungsbehälters abgezogene Absorptionsflüssigkeit wurde schließlich nach Durchlaufen eines Wärmetauschers in einen dritten Entspannungsbehälter entspannt, in dem mittels eines Wasserdampfstrahlers ein Druck von 0,7 bar aufrechterhalten wurde. Die in diesem Entspannungsbehälter freigesetzte $CO_2$-Menge betrug 0,08 kmol/h. Das am Kopf des dritten Entspannungsbehälters abgezogene Gas wurde zusammen mit dem Wasserdampf, mit dem der Wasserdampfstrahler betrieben wurde, in den Sumpf des zweiten Entspannungsbehälters eingeleitet. Am Kopf des zweiten Entspannungsbehälters wurden 0,18 kmol/h eines $CO_2$-reichen Entspannungsgases mit einer $CO_2$-Konzentration von mehr als 98 Mol.-% abgezogen. Die am Sumpf des dritten Entspannungsbehälters abgezogene Absorptionsflüssigkeit wurde mit Hilfe einer Umlaufpumpe zum Kopf der Absorptionskolonne zurückgepumpt.

Patentansprüche

1. Verfahren zum Entfernen von $CO_2$ und/oder $H_2S$ aus $CO_2$ und/oder $H_2S$ enthaltenden Gasen durch eine Wäsche der Gase mit einer wäßrigen, ein Alkanolamin enthaltenden Absorptionsflüssigkeit, dadurch gekennzeichnet, daß man die $CO_2$ und/oder $H_2S$ enthaltenden Gase in einer Absorptionsstufe mit einer 3 bis 60 Gew.% eines tertiären Alkanolamins und 1 bis 50 Gew.% eines primären Alkanolamins enthaltenden wäßrigen Absorptionsflüssigkeit behandelt, am Kopf der Absorptionsstufe die behandelten Gase abzieht, am Sumpf der Absorptionsstufe die mit $CO_2$ und/oder $H_2S$ beladene wäßrige Absorptionsflüssigkeit abzieht und zur Regenerierung in einer oder mehreren Entspannungsstufen entspannt und die regenerierte Absorptionsflüssigkeit in die Absorptionsstufe zurückführt.

2. Verfahren nach Anspruch 1, dadurch gekennzeichnet, daß man die mit $CO_2$ und/oder $H_2S$ beladene wäßrige Absorptionsflüssigkeit zur Regenerierung in mindestens zwei Entspannungsstufen entspannt und dabei die letzte Entspannungsstufe bei vermindertem Druck gegenüber Atmosphärendruck betreibt.

3. Verfahren nach Anspruch 2, dadurch gekennzeichnet, daß man zum Ausgleich der Wasserverluste durch in den am Kopf der Absorptionsstufe und den Entspannungsstufen abgezogenen Gasströmen enthaltenes Wasser am Sumpf der vorletzten Entspannungsstufe eine dem Wasserverlust entsprechende Menge Wasserdampf zuführt.

4. Verfahren nach Anspruch 2 und 3, dadurch gekennzeichnet, daß der verminderte Druck in der letzten Entspannungsstufe mittels eines Wasserdampfstrahlers erzeugt wird.

5. Verfahren nach Anspruch 4, dadurch gekennzeichnet, daß das am Kopf der letzten Entspannungsstufe abgezogene Gas zusammen mit dem Wasserdampf, mit dem der Wasserdampfstrahler betrieben wird, am Sumpf der vorletzten Entspannungsstufe zugeführt wird.

6. Verfahren nach Anspruch 1 bis 5, dadurch gekennzeichnet, daß man die Absorptionsflüssigkeit verwendet, die 3 bis 60 Gew.% Methyldiethanolamin und 1 bis 50 Gew.% Monoethanolamin enthält.

Zeichn.

O.Z. 0050/37235
0172408

7/2

FIG.1

O.Z. 0050/37235

FIG.2